# EUROPEAN PATENT SPECIFICATION

(11) **EP 2 204 204 B1**
(45) Date of publication and mention of the grant of the patent: **06.09.2017**
(21) Application number: 09405229.7
(22) Date of filing: 21.12.2009
(51) Int. Cl.: A61M 5/32, A61M 25/06

(54) **Needle protection device**
Nadelschutzvorrichtung
Dispositif de protection d'aiguille

(30) Priority: 30.12.2008 CH 20262008
(43) Date of publication of application: 07.07.2010
(73) Proprietor: Tipromed S.r.l., 42041 Sorbolo a Levante di Brescello (RE) (IT)
(72) Inventor: Petralli, Franco, 6900 Lugano (CH)
(74) Representative: Negrini, Elena

(56) References cited:
- WO-A1-03/011381
- US-A- 5 344 408
- US-A1- 2004 049 163
- US-A1- 2004 186 434
- US-A1- 2008 243 086
- US-B1- 6 203 527

## Description

The present invention relates to the technical field of medical products provided with a needle, in particular vein catheters with introducer needle which are provided with a needle safety device of the passive type.

The present invention refers to a needle protection device of a generic medical instrument, as described in the preamble of claim 1.

The subject of the invention, the needle protection device of a generic medical instrument, allows the cutting apex of the needle to be covered and to be locked inside a container, as the needle is retracted from the blood vessel after having carried out its piercing function.

Commonly used medical products provided with a needle lack in specific protection for the needle. In the following the prior art will be described.

Referring to a needle-catheter device, the operator inserts the needle-catheter assembly by piercing the epidermis to reach a patient's vein or artery. Once the insertion has been carried out and completed, the catheter is kept in place inside the blood vessel, while the needle, having carried out its piercing function, is extracted by the operator. In this critical step, the sharp tip of the extracted needle remains exposed causing a danger to the operator or to a person nearby. The risk of inadvertent pricks is high. The operation of inserting the needle into a protection casing for its disposal is equally dangerous to the operator.

Devices are also known which provide for the retraction of the needle into a protection element, e. g. a portion of the device itself, once the catheter has been inserted. A device according to the preamble of claim 1 is disclosed in US2008/0243086. The retraction is effected by releasing an elastic means, that thrusts the needle into a disposal compartment by acting on a sleeve on the outside of the catheter, to which the needle is rigidly connected and whose axial sliding determines the needle retraction.

The main drawback of such devices lies in the dimensions of the protection element. Another drawback is that such protection devices are characterized by a large number of components, which makes their assembly complicated.

Vein and intravenous catheters with closed system are also known which comprise a flexible plastic tube wherein an introducer needle or metal mandrel is housed, whose cutting tip projects form the catheter apex.

Also in the case of generic medical devices provided with a needle, after the extraction of the latter from a patient's body, the apical cutting edges remain dangerously exposed, causing the risk of inadvertent pricks to the operator.

Severe diseases such as hepatitis or AIDS can be transmitted due to the prick of a blood dirty needle extracted from a patient affected by such pathologies.

An object of the invention is to provide a protection device for a needle that avoids the dangers of infective diseases transmission due to inadvertent pricks with a needle soiled with blood, thus allowing a totally secure disposal of the retracted needle.

Another object is to provide a device which can be easily fabricated and assembled, and thus is inexpensive.

Another object is to provide a device which is safe and has a smooth needle retraction which avoids blood splashes as well as contaminations caused by particles generated by a scraping action of the locking elements against the needle while the latter is being retracted.

Another object of the invention is to provide a protection device requiring a very small activation force.

The above-mentioned objects are achieved by means of the needle protection device described in claim 1.

Further advantageous developments of the needle protection device of the invention are provided in the dependent claims.

The invention is now better illustrated with reference to the accompanying drawings, which schematically show preferred embodiments thereof only for indicating and non-limiting purposes, since it will be always possible to introduce technical or construction variants without departing from the scope of the present invention.

In said drawings:
Figure 1A shows an embodiment of the needle protection device in a non-operating condition I, in a lateral view, sectioned along the needle axis;
Figure 1B shows the device of Figure 1A in the same view, but in an operating condition II;
Figure 1C shows the device of Figure 1B in the same view, with needle rotated by 180°, in the operating condition II;
Figure 1D shows in a front view the cross-section of the container according to the present invention, in the region of the central cavity C2, without the protection means;
Figure 2 shows an embodiment of the needle protection device fitted to a medical instrument MED, in particular a closed system with vein catheter;
Figure 3 shows in a lateral view a component of the invention: the protection means 4;
Figure 4 shows in a lateral view an embodiment of the protection device 1 in the operating condition II;
Figure 5A shows in an exploded view an embodiment of the protection device 1 fitted to a medical instrument MED;

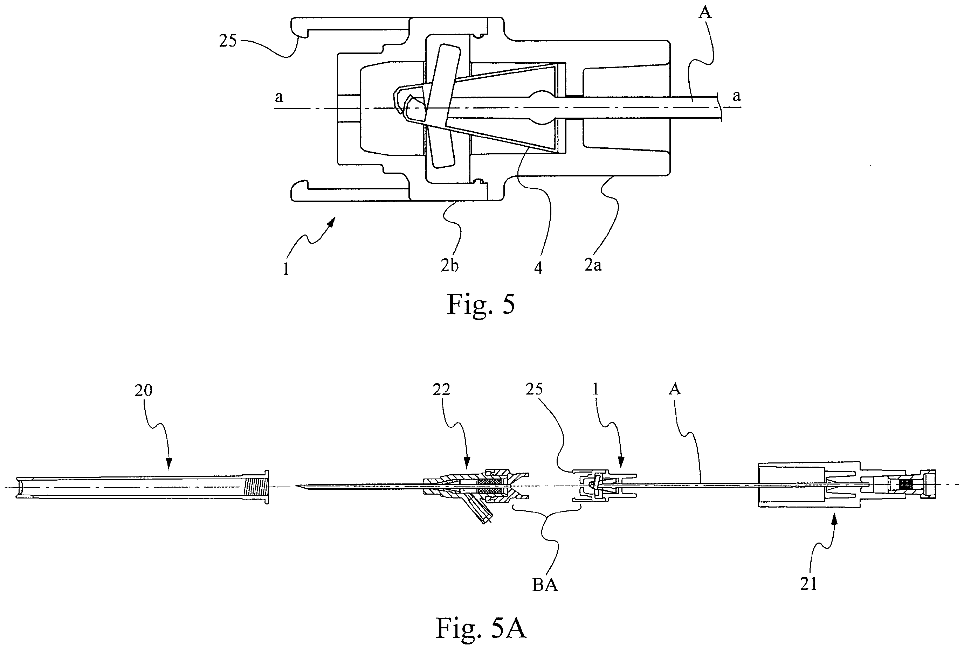

Figure 5 shows in a lateral view an embodiment of the needle protection device 1;

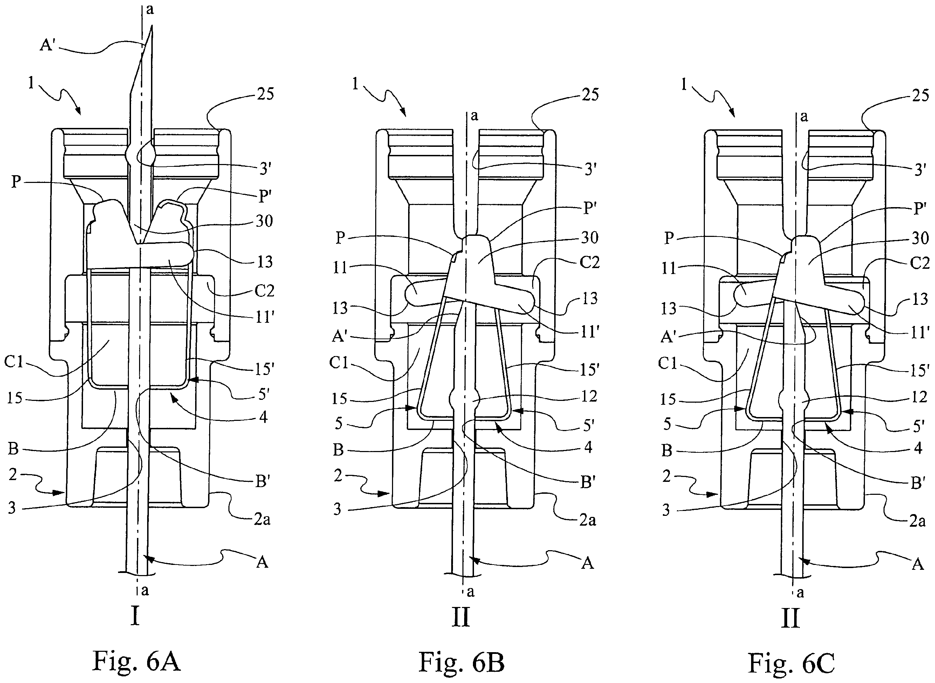

Figure 6A shows a second embodiment of the needle protection device in the non-operating condition I, in a lateral view, sectioned along the needle axis;
Figure 6B shows the device of Figure 6A in the same view, but in the operating condition II;
Figure 6C shows the device of Figure 6B in the same view, with needle rotated by 180°, in the operating condition II;

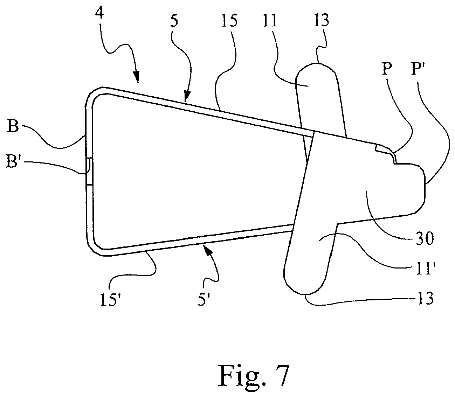

Figure 7 shows in a lateral view in place an alternative embodiment of a component of the invention: the protection means 4; and

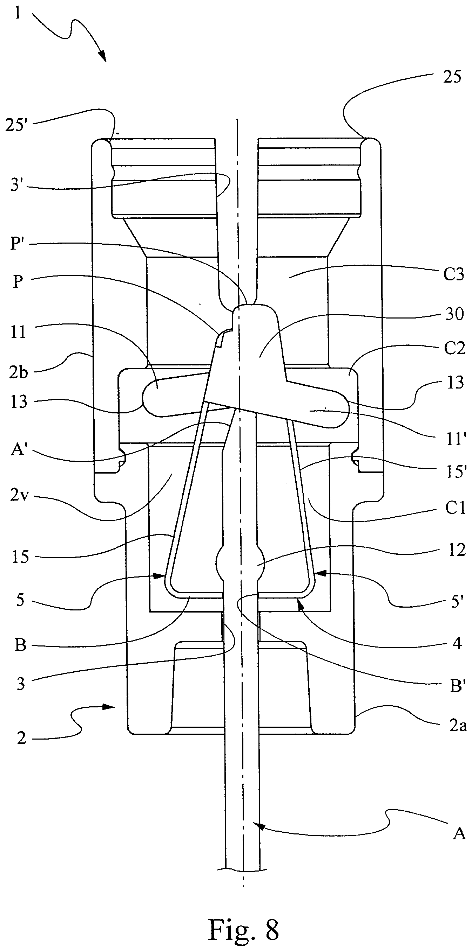

Figure 8 shows in an enlarged lateral view the embodiment of figure 6A of the protection device 1 in the operating condition II.

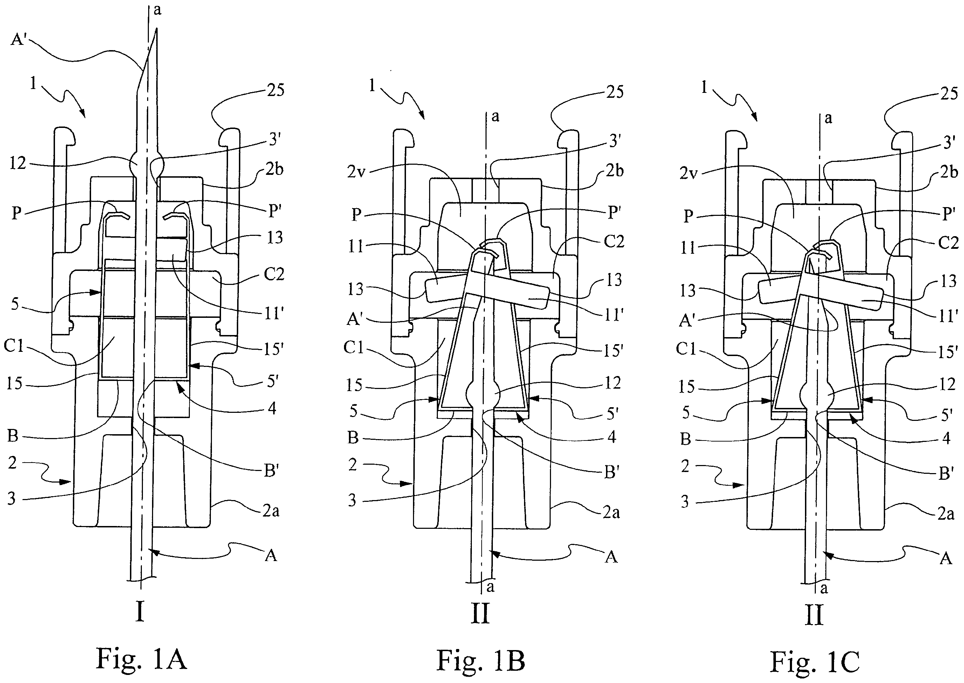

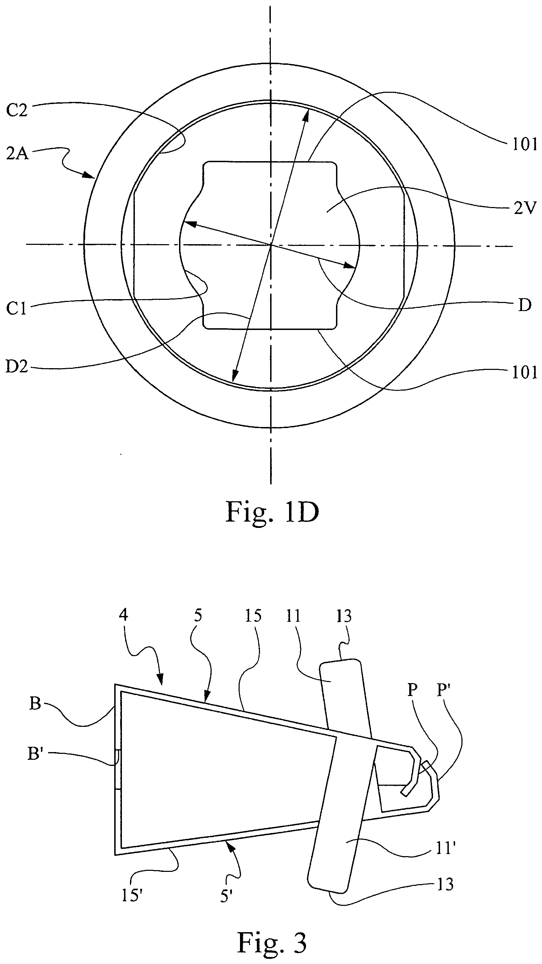

Referring to Figures 1A, 1B e 1C, the needle protection device 1 of a generic medical instrument, which is the subject of the invention, comprises a container 2 provided with through holes 3, 3' adapted to allow the sliding of the needle A, and a protection means 4 movably housed inside said container 2. Said container 2 has substantially a cylindrical shape, as it can be seen in Figure 1D.

The container 2 consists of a housing 2a removably assembled to a cap 2b. The housing 2a and the cap 2b are joined by means of joint, in particular a concentric annular joint of the mechanical type.

Inside the container 2, a compartment 2v is formed which contains the protection means 4 and a portion of the needle A.

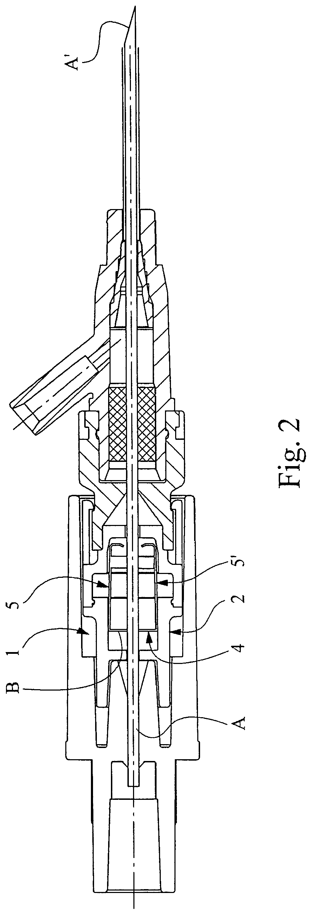

The needle protection device 1 comprises a container 2 provided with concentric through holes 3, 3' adapted to allow the needle A to axially slide inside the container 2. The through holes 3, 3' are provided at axially opposite ends of the container, in corresponding and axially aligned positions. In detail, the through hole 3 is provided in the housing 2a, while the through hole 3' is provided in a corresponding position in the cap 2b. In the embodiment shown in figures 1-5 the through holes 3, 3' have substantially the same diameter adapted to allow the passage of the needle A.

In the present invention, the expression "axial" and/or "axially" refers to a direction parallel to the direction defined by the vertical symmetry axis of the needle.

As previously mentioned, the device 1 further comprises a protection means 4 movably housed inside the contained 2.

To this end, as better shown in figure 1D, the container 2 and in particular the inner portion of the housing 2a has a slot 101 adapted to receive axially slidably in axial direction a base of the protection element 4.
The protection means 4 is further provided with locking members 5, 5' and projection 11, 11' in form of wings 11, 11'.

According to an advantageous aspect of the present invention the locking members 5, 5' are adapted to cover the sharp end A' of the needle.
In detail, the protection means 4 is provided with locking members 5, 5' adapted to cover the sharp end A' of the needle and to lock the same inside the protection means, when the device is activated from a non-operating condition I (figure 1A) to an operating condition II (figure 1B).

Advantageously, in one embodiment thereof shown in the figures 1-5, the protection means 4 which, in the operating condition II, locks the sharp end of the needle A is an elastic plate, preferably made of metal, shaped in a triangular form and provided with a base B and two locking members 5, 5' extending oppositely to each other from the base B, as shown in figure 3.

The locking members 5, 5' can be defined by two jaws 15, 15' flexible upon compression and each provided with an apex P, P' curved towards the inside of the protection means 4.

Advantageously, the locking members 5, 5' have a length different from each other, so that in the operating condition II the two curved apexes P, P' can overlap enclosing the sharp end A' of the needle A. The base of the plate B and the two jaws 15, 15' form a single element, while the apexes P, P' of the two jaws 15, 15' are distinct.

In particular, the shaping of the plate forming the protection element 4 defines a first jaw 15 and a second jaw 15' having different heights, adapted to enclose the sharp tip A' of the needle A when the device is in the operating condition II.

When the device is activated - operating condition II - the jaws 15, 15', due to the elastic force, bring near to each other their apexes P, P' and made them to overlap, so as to cover the needle, particularly the sharp portion A'.

Form the locking members 5, 5' also extend projections in the form of wings 11, 11', which are adapted to get wedged, in the operating condition II, into an inner cavity of the container 2, positioned between the two through holes 3, 3', for preventing the device from reverting from the operating condition II to the non-operating condition I and thus a dangerous extraction of the needle A.

The two wings 11, 11' are spaced apart and positioned at transversally outer edges of the said locking members 5, 5'.

In the present description, the expression "transversal direction" or "transversally" indicates a direction substantially perpendicular to the axial direction.

Preferably, there is provided one wing 11, 11' for each jaw 15, 15'. In detail, each wing 11, 11' is rigidly connected to the transversally outer end of a jaw 15, 15' and projects substantially perpendicularly therefrom, so as to face the remaining wing. The two wings 11, 11' are arranged at a predetermined distance from the larger base B of the plate. Preferably, the wings 11, 11', at least close to the respective jaw 15, 15' with which they are associated, are positioned at the same distance from the larger base B of the plate.

Preferably, the wings 11, 11' are positioned close to the apexes P, P' of the two laws 15, 15'. In any case, they are positioned at a height greater than at least 40% of the height of the two jaws 15; 15'.

Each wing 11, 11' is dimensioned and shaped for projecting form the respective jaw 15, 15' to such an extent that in the non-operating condition I each free end 13 of the wing (i. e. the one not linked to the jaw) abuts against an inner wall of the container 2 for bending the jaws 15, 15' and moving the curved apexes P, P' apart from each other at a distance that allows the needle A to move between them without rubbing, as shown for example in figure 1A.
The needle protection device 1 is characterized in that it can be activated, by means of a displacement of the protection means 4 relative to the container 2, from a non-operating condition I (shown in figure 1A), in which the needle A is freely movable inside the container 2, to an operating condition II (shown in the figures 1B, 1C), in which said locking members 5, 5' interact with the container 2 so as to: wedge said projections 11, 11' into an inner cavity of the container 2, positioned between the two through holes 3, 3', and cover the sharp end A' of the needle.

The activation of the needle protection device 1 is determined by the axial displacement of the needle A further to its extraction from the vein.
To this end, the needle A is provided on its cylindrical body with a permanent deformation 12, that increases the diameter of the needle A. The permanent deformation 12 is adapted to interact with the base of the elastic plate B, and in particular with a through hole B' so as to allow the dragging of the protection means 4 by means of an interference.
In the present case the permanent deformation 12 has a substantially ovoid or spherical shape adapted to engage with the base of the elastic plate so as to cause a secure interference deformation/hole. For this purpose, the diameter of the permanent deformation 12 is larger than the diameter of the through hole B' formed at the base of the protection means 4.

The needle protection device 1 can be activated by means of a displacement of the protection means 4 relative to the container 2, from a non-operating condition I - in which the needle is retracted from the vein - to an operating condition II in which the locking members 5, 5' interact with the container 2 so as to cover the sharp end A' of the needle A, locking the same inside the container 2, in the present case the ends of the locking members engage with each other.

As previously mentioned, the protection means 4, together with the needle A, is axially slidable inside the container 2.

To this end, the container 2 defines in its interior three cylindrical, concentric cavities C1, C2, C3 adjacent to one another, of which those C1, C3 at the two ends have a rectangular slot 101 adapted to receive the protection means 4 allowing the axial sliding thereof.

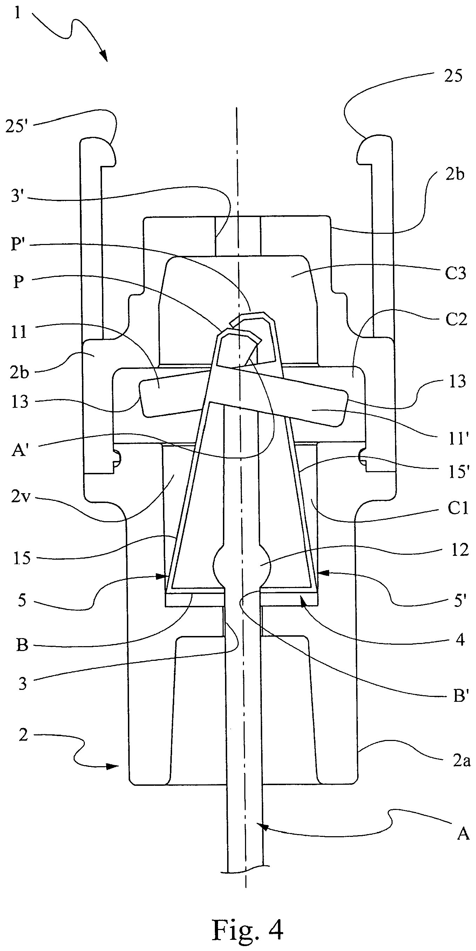

As better apparent in figure 4, the three cavities C1, C2 and C3 define the compartment 2v for housing the protection means 4 and allowing the sliding thereof.

Preferably, two C1, C3 of the three cavities C1, C2, C3 have an equal diameter, while the cavity C2 positioned between the two end cavities C1, C2 has a larger diameter than the others.
The central cylindrical cavity C2 has a larger diameter than that of the two ends C1, C2, so as to be adapted to receive the wings 11, 11' in the operating position II (fig. 1B), thus blocking the stroke of the protection means 4 and allowing the locking members 5, 5' to cover the sharp end A' of the needle.

Passing from the non-operating position I to the operating position II the protection means 4 slides axially inside the compartment 2v together with the needle A.

The two wings 11, 11' also slide axially passing from the cavity C3 into the cavity C2 with larger diameter. In fact, the diameter of the cavity C3 prevents the elastic recovery of the two jaws 15, 15', forcing the curved apexes P, P' of the two jaws 15, 15' apart.
In the cavity C2 of larger size, the jaws 15, 15' are no longer forced apart by the interaction between the wings 11, 11' and the inner walls of the cavity C3 and thus return elastically in a rest position, in which they bring near to each other their apexes P, P' and make them to overlap, enclosing at the same time the sharp end A' of the needle A.

When the wings 11, 11' reach the cavity C2, the device is in the operating condition II, the protection means 4 has retracted itself and gets wedged in the container 2 so that its stroke can no longer continue.

The wedging occurs by means of the wings 11, 11' of the protection means 4. In detail, the free end portions 13 of the wings 11, 11' enter the cavity portion C2 having a diameter larger than the diameter of the cavities C1, C3, and get locked therein.

Any axial movement of the needle A makes the free ends 13 of the wings 11, 11' to abut against the walls of the cavity C2 without the possibility of overcoming them.

Thus, in the operating condition II the sharp tip A' of the needle is covered by the protection means 4 and cannot come out from said protection means 4 being locked by the locking members 5, 5'.

Advantageously, in the embodiment shown in figures 1-5 the protection device 1 is provided with pincer-like connections 25 suitable for automatic release of the protection device from the body of the catheter 22.
In figures 6-8 a further embodiment of the device according to the present invention is shown.
The container 2 is provided also in this embodiment with a first through hole 3 and a second through hole 3', in which the needle A is positioned in a slidable/movable way. Said through holes are concentric with each other.
However, in this embodiment the upper hole 3' has a larger diameter than the hole 3.

The container 2 consists of two cylindrical bodies, in particular a housing 2a and a cap 2b removably assembled with each other.

Inside the container 2 a compartment 2v is formed which contains the protection means 4 and a portion of the needle A.
In the embodiment shown in the figures 6-8 the compartment 2v of the container 2 comprises at least three cylindrical, concentric cavities C1, C2, C3 adjacent to one another.

The central cylindrical cavity C2 has larger diameter larger that the diameter of the two end C1, C2, so as to receive and lock the wings 11, 11'.

Also in this embodiment, by blocking the axial stroke of the wings 11, 11' the stroke of the protection means is also blocked and the locking members 5, 5' can cover the sharp end of the needle A'.

Advantageously, in such an embodiment, the protection means 4 which prevents the extraction of the needle A is an elastic plate, preferably made of metal, shaped in triangular form and provided with a base B and two locking members 5, 5', in particular two elastic jaws 15, 15' provided at their upper portions with two inwardly curved apexes P, P'.

The two jaws 15, 15' extend from the base B facing each other at predetermined distance.

Preferably, the two jaws 15, 15' have different heights and are shaped so as to enclose the sharp tip A' of the needle A when the device 1 is in the operating condition II.

When the device 1 is activated - operating condition II - the jaws 15, 15' bring near to each other their apexes P, P' and made them to overlap, so as to cover the needle A, particularly the sharp portion A'.

Form the locking members 5; 5' also extend projections in the form of wings 11, 11' adapted to get wedged, in the operating condition II, into an inner cavity of the container 2 positioned between the two through holes 3, 3' for preventing the device 1 from reverting from the operating condition II to the non-operating condition I and thus a dangerous extraction of the needle A.

The two wings 11, 11' are transversally spaced apart and positioned at outer ends of the jaws 15, 15'.

Preferably, also in this case there is provided one wing 11, 11' for each jaw 15, 15'. In detail, each wing 11, 11' is rigidly connected to the axially outer end of a jaw 15, 15' and projects substantially perpendicularly therefrom, so as to face the remaining wing. The two wings 11, 11' are positioned at a predetermined distance from the larger base B of the plate. Preferably, the wings 11, 11', at least close to the respective jaw with which they are associated, are positioned at the same distance from the larger base B of the plate. Preferably, the wings 11, 11' are positioned close to the apexes P, P' of the two laws 15, 15'. In any case, they are positioned at at least 40% of the height of the two jaws 15; 15'.

Advantageously, the embodiment shown in figures 6-8 has at least one cover element 30, positioned between a wing 11, 11' and a curved apex P, P' of the aforementioned jaw 15, 15' for preventing the needle from coming out laterally in the operating condition (II).

In detail, in the embodiment shown in the figures there are two spaced apart cover elements 30, each positioned at the transversally outer end of a jaw 15, 15' between a wing 11, 11' and the curved apex P, P' of the same jaw for enclosing such space and prevent the possible attempt of the needle to come out laterally in the operating condition (II).

Each cover element 30 is rigidly connected to a wing 11, 11' and is made by the same plate forming the bent jaw. As shown in the figures, the cover element 30 is made by bending the plate of the jaw so that the cover element 30 is coplanar with the wing rigidly connected thereto and substantially perpendicular to the curved apex P, P' and to the jaw itself.

In the base of the elastic plate, at the center thereof, a through hole B' is formed having a diameter slightly larger than that of the needle A.

The needle A is provided on its cylindrical body with a permanent deformation 12, that increases the diameter of the needle A. The permanent deformation 12 is adapted to interact with the base B of the elastic plate, and in particular with the through hole B' so as to allow the dragging of the protection means 4 by means of an interference.
In the present case the permanent deformation 12 has a substantially ovoid or spherical shape adapted to engage with the base of the elastic plate so as to cause a secure interference deformation/hole. The diameter of the permanent deformation 12 is in fact larger than the diameter of the through hole B' formed at the base of the protection means 4.

The dragging of the protection means 4 causes the activation of the protection device 1: the wings get wedged in the central cylindrical cavity C2 thus blocking the dragging of the protection element 4. When the protection device is not active - non-operating position I (fig. 6A) - the wings 11, 11' are elastically compressed and aligned with the base B of the plate 4. The resulting shape is a parallelogram partially lacking one side, with the apexes P, P' of the jaws 15, 15' not overlapping, but spaced form the needle so as to allow the passage of the needle A between them, without rubbing on the same. It shall be remarked that a main advantage of the finding is the absence of any rubbing of the outer surface of the needle on one or more component of the protection device. While the needle A moves back, the deformation 12 on a portion of the needle A by interacting with the hole B' causes the protection means 4 to move back into the device.

The protection means 4 moves back as long as the free ends of the wings 11, 11' reach the cavity C2 with larger diameter. There the free end portions 13 of the wings 11, 11' enter the portion of the cavity C2 having a diameter larger than the diameter of the cavities C1, C3, and get locked therein.
Any axial movement of the needle A makes the free ends 13 of the wings 11, 11' to abut against the walls of the cavity C2 without the possibility of overcoming them.

In the cavity C2 of larger size, the jaws 15, 15' are no longer forced apart by the interaction between the wings 11, 11' and the inner walls of the cavity C3 and thus return elastically in a rest position, in which they bring near to each other their apexes P, P' making them to overlap and enclose the sharp end A' of the needle A.

Advantageously, according to this embodiment, thanks to the presence of the cover elements 30 in the operating condition (II), the possible attempt of the needle to come out laterally is prevented.

The main advantage of the invention is to provide a protection device for a medical device provided with a needle, which protects against the risk of contact with the substance by which the needle has been soiled and against inadvertent injuries.

The protection device subject of the invention is of the passive type and does not require an activation by the operator when mounted on vein catheters. Moreover, the inventive device can be easily fabricated and assembled.

## Claims

1. Needle protection device (1) of a generic medical instrument, wherein a needle with a cylindrical body having a first diameter and provided with an enlarged portion (12) having a diameter larger than the first diameter of the cylindrical body is fitted to said generic medical instrument, said device (1) comprises: a container (2) provided with concentric through holes (3, 3') adapted to allow the needle (A) to slide inside the container (2), a protection means (4) movably housed inside the container (2), said protection means (4) being provided with a base (B) having a hole (B') with a diameter such that the cylindrical body of the needle (A) having the first diameter can slide through the hole (B') and two locking members (5, 5') extending oppositely to each other from the base (B), the locking members (5, 5') defined by two jaws flexible upon compression and each provided with an apex (P, P') and with a projection (11, 11') in the form of a wing; the locking members (5, 5') adapted to cover the sharp end of the needle (A), **characterized in that** said device (1) can be activated, by means of an axial displacement of the protection means (4) relative to the container (2), from a non-operating condition I, in which the needle (A) is freely movable inside the container (2), to an operating condition II, in which said locking members (5, 5') interact with the container (2) so as to: wedge said projections (11, 11') into a cavity of the container (2) positioned between the two through holes (3, 3') for preventing the device from reverting from the operating condition II to the non-operating condition I and cover the sharp end (A') of the needle;
- the diameter of the hole (B') of the base (B) is smaller than the diameter of the enlarged portion (12), such that when the protection device (1) is moved relative to the needle to cover the sharp end of the needle (A), the enlarged diameter portion (12) can get into contact with the base (B) to drag the protection means (4) axially relative to the container (2);
- each wing is dimensioned and shaped for projecting from the respective jaw to such an extent that in the non-operating condition I each free end of the wing abuts against an inner wall of the container for bending the jaws and moving the apexes (P, P') apart from each other at a distance that allows the needle A to move between them without rubbing, and each jaw (15, 15') is configured for returning elastically to a position where its apex (P, P') covers the sharp end of the needle (A) when its projection is wedged in the cavity of the container (2) in the operating condition II.

2. Needle protection device of a generic medical instrument according to claim 1, **characterized in that** the protection means (4) is a metal plate.

3. Needle protection device (1) of a generic medical instrument according to one of the previous claims, **characterized in that** the locking members (5, 5') are two flexible jaws (15, 15') each provided with an inwardly curved apex (P, P').

4. Needle protection device (1) of a generic medical instrument according to anyone of the previous claims, **characterized in that** the locking members (5, 5') have a length different from each other.

5. Needle protection device (1) of a generic medical instrument according to claim 1, **characterized in that** said projections (11; 11') comprise two wings spaced apart and positioned at transversally outer edges of the said locking members (5, 5').

6. Needle protection device (1) of a generic medical instrument according to claim 5, **characterized in that** said two wings are positioned at a predetermined distance from a base of the protection means (4) at at least 40% of the height of the said locking members (5; 5').

7. Needle protection device (1) of a generic medical instrument according to claim 5, **characterized in that** each wing is rigidly connected to the axially outer end of a locking member (5, 5') and projects substantially perpendicularly therefrom.

8. Needle protection device (1) of a generic medical instrument according to anyone of the previous claims, **characterized by** comprising at least one cover element (30), positioned between a projection and a curved apex (P; P') of the said locking member (5, 5') for preventing the needle from coming out laterally in the operating condition (II).

9. Needle protection device (1) of a generic medical instrument according to claim 8, **characterized by** comprising two spaced apart cover elements (30), each being positioned at the transversally outer end of a locking member (5; 5') between a projection and a curved apex (P, P') of the said locking member (5, 5') for preventing the needle from coming out laterally in the operating condition (II).

10. Needle protection device (1) of a generic medical instrument according to claim 8 or 9, **characterized in that** each cover element (30) is rigidly connected a wing.

11. Needle protection device (1) of a generic medical instrument according to anyone of the previous claims, **characterized in that** the container (2) comprises at least three cylindrical, concentric cavities (C1, C2, C3) adjacent to one another.

12. Needle protection device (1) of a generic medical instrument according to claim 11, **characterized in that** two (C1, C3) of the three cavities (C1, C2, C3) have a same diameter and **in that** the cavity (C2) positioned between the two ends (C1, C3) has a larger diameter than the diameters of the cavities positioned at the two ends.

13. Needle protection device (1) of a generic medical instrument according to claim 12, **characterized in that** at least one cavity has a slot (101) for at least partially housing the protection means (4).

14. Needle protection device (1) of a generic medical instrument according to one of the previous claims, **characterized in that** in the non-operating condition (I) the distance between the curved apexes (P, P') is larger than the diameter of the needle (A).

15. Needle protection device (1) of a generic medical instrument according to one of the previous claims, wherein the needle (A) is fitted to a vein catheter.

## Patentansprüche

1. Nadelschutzvorrichtung (1) eines generischen medizinischen Instruments, wobei eine Nadel mit einem zylindrischen Körper, der einen ersten Durchmesser aufweist und mit einem erweiterten Bereich (12) ausgestattet ist, der einen größeren Durchmesser als der erste Durchmesser des zylindrischen Körpers aufweist, an das generische medizinische Instrument angepasst ist, wobei die Vorrichtung umfasst:
einen Behälter (2), der bereitgestellt ist mit konzentrischen Durchgangslöchern (3, 3'), die geeignet sind, der Nadel (A) zu gestatten, in den Behälter (2) hinein zu gleiten,
ein Schutzmittel (4), das im Inneren des Behälters (2) bewegbar angeordnet ist, wobei das Schutzmittel (4) mit einer Basis (B) bereitgestellt ist, die aufweist ein Loch (B') mit einem Durchmesser, sodass der zylindrische Körper der Nadel (A) mit dem ersten Durchmesser durch das Loch (B') gleiten kann, und zwei Sperrglieder (5, 5'), die sich einander gegenüberliegend von der Basis (B) erstrecken, wobei die Sperrglieder (5, 5') definiert sind durch zwei Backen, die bei Zusammendrücken flexibel sind und jeweils mit einem Scheitel (P, P') und mit einem Vorsprung (11, 11') in Form eines Flügels bereitgestellt sind,
wobei die Sperrglieder (5, 5') angepasst sind, das scharfe Ende der Nadel (A) abzudecken, und **dadurch gekennzeichnet, dass**
die Vorrichtung (1) aktiviert werden kann mit Hilfe eines axialen Versatzes des Schutzmittels (4) in Bezug auf den Behälter (2) aus einem Nicht-Betriebszustand I, in dem die Nadel (A) im Inneren des Behälters (2) frei bewegbar ist, in einen Betriebszustand II, in dem die Sperrglieder (5, 5') mit dem Behälter (2) zusammenwirken, um die Vorsprünge (11, 11') in einem Hohlraum des Behälters (2) zu verkeilen, der zwischen den beiden Durchgangslöchern (3, 3') positioniert ist, um zu verhindern, dass die Vorrichtung aus dem Betriebszustand II in den Nicht-Betriebszustand I zurückkehrt, und um das scharfe Ende der Nadel (A') abzudecken,
wobei der Durchmesser des Lochs (B') der Basis (B) kleiner ist als der Durchmesser des erweiterten Bereichs (12), sodass, wenn die Schutzvorrichtung (1) in Bezug auf die Nadel bewegt wird, um das scharfe Ende der Nadel (A) abzudecken, der Bereich (12) mit dem erweiterten Durchmesser mit der Basis (B) in Kontakt kommen kann, um das Schutzmittel (4) in Bezug auf den Behälter (2) axial zu ziehen, wobei
jeder Flügel so dimensioniert und geformt ist, um von der jeweiligen Backe in einem solchen Ausmaß vorzustehen, dass in dem Nicht-Betriebszustand I jedes freie Ende des Flügels an einer inneren Wand des Behälters anliegt, um die Backen zu biegen und die Scheitel (P, P') voneinander weg in eine Entfernung zu bewegen, die es der Nadel A gestattet, sich zwischen ihnen ohne Reibung zu bewegen, und jede Backe (15, 15') ist konfiguriert, um elastisch in eine Position zurückzukehren, in der ihr Scheitel (P, P') das scharfe Ende der Nadel (A) abdeckt, wenn ihr Vorsprung im Betriebszustand II im Hohlraum des Behälters (2) verkeilt ist.

2. Nadelschutzvorrichtung eines generischen medizinischen Instruments gemäß Anspruch 1, **dadurch gekennzeichnet, dass** das Schutzmittel (4) eine Metallplatte ist.

3. Nadelschutzvorrichtung (1) eines generischen medizinischen Instruments gemäß einem der vorhergehenden Ansprüche, **dadurch gekennzeichnet, dass** die Sperrglieder (5, 5') zwei biegsamen Backen (15, 15') sind, die jeweils mit einem nach innen gebogenen Scheitel (P, P') bereitgestellt sind.

4. Nadelschutzvorrichtung (1) eines generischen medizinischen Instruments gemäß einem der vorhergehenden Ansprüche, **dadurch gekennzeichnet, dass** die Sperrglieder (5, 5') unterschiedliche Längen aufweisen.

5. Nadelschutzvorrichtung (1) eines generischen medizinischen Instruments gemäß Anspruch 1, **dadurch gekennzeichnet, dass** die Vorsprünge (11, 11') zwei Flügel umfassen, die beabstandet sind und an querverlaufenden Außenrändern der Sperrglieder (5, 5') angeordnet sind.

6. Nadelschutzvorrichtung (1) eines generischen medizinischen Instruments gemäß Anspruch 5, **dadurch gekennzeichnet, dass** die beiden Flügel in einem vorgegebenen Abstand von einer Basis des Schutzmittels (4) bei mindestens 40 % der Höhe der Sperrglieder (5, 5') angeordnet sind.

7. Nadelschutzvorrichtung (1) eines generischen medizinischen Instruments gemäß Anspruch 5, **dadurch gekennzeichnet, dass** jeder Flügel fest mit dem axial äußeren Ende eines Sperrglieds (5, 5') verbunden ist und sich im Wesentlichen senkrecht davon erstreckt.

8. Nadelschutzvorrichtung (1) eines generischen medizinischen Instruments gemäß einem der vorhergehenden Ansprüche, **dadurch gekennzeichnet, dass** es mindestens ein Abdeckelement (30) aufweist, das zwischen einem Vorsprung und einem gebogenen Scheitel (P, P') des Sperrglieds (5, 5') angeordnet ist, um zu verhindern, dass die Nadel seitlich in den Betriebszustand (II) heraus gelangt.

9. Nadelschutzvorrichtung (1) eines generischen medizinischen Instruments gemäß Anspruch 8, **dadurch gekennzeichnet, dass** sie zwei beabstandete Abdeckelemente (30) umfasst, wobei jedes am querverlaufenden äußeren Ende eines Sperrglieds (5, 5') zwischen einem Vorsprung und einem gebogenen Scheitel (P, P') des Sperrglieds (5, 5') angeordnet ist, um zu verhindern, dass die Nadel seitlich in den Betriebszustand (II) heraus gelangt.

10. Nadelschutzvorrichtung (1) eines generischen medizinischen Instruments gemäß Anspruch 8 oder 9, **dadurch gekennzeichnet, dass** jedes Abdeckelement (30) fest mit einem Flügel verbunden ist.

11. Nadelschutzvorrichtung (1) eines generischen medizinischen Instruments gemäß einem der vorhergehenden Ansprüche, **dadurch gekennzeichnet, dass** der Behälter (2) mindestens drei zylindrische, konzentrische Hohlräume (C1, C2, C3) aufweist, die benachbart zueinander sind.

12. Nadelschutzvorrichtung (1) eines generischen medizinischen Instruments gemäß Patentanspruch 11, **dadurch gekennzeichnet, dass** zwei (C1, C3) der drei Hohlräume (C1, C2, C3) einen gleichen Durchmesser aufweisen und dadurch, dass der Hohlraum (C2), der zwischen den beiden Enden (C1, C3) angeordnet ist, einen größeren Durchmesser als die Durchmesser der an den beiden Enden angeordneten Hohlräume aufweist.

13. Nadelschutzvorrichtung (1) eines generischen medizinischen Instruments gemäß Anspruch 12, **dadurch gekennzeichnet, dass** mindestens ein Hohlraum einen Schlitz (101) aufweist, um das Schutzmittel (4) zumindest teilweise aufzunehmen.

14. Nadelschutzvorrichtung (1) eines generischen medizinischen Instruments gemäß einem der vorhergehenden Ansprüche, **dadurch gekennzeichnet, dass** der Abstand zwischen den gebogenen Scheiteln (P, P') im Nicht-Betriebszustand (I) größer ist als der Durchmesser der Nadel (A).

15. Nadelschutzvorrichtung (1) eines generischen medizinischen Instruments gemäß einem der vorhergehenden Ansprüche, wobei die Nadel (A) an einen Venenkatheter angepasst ist.

## Revendications

1. Dispositif de protection d'aiguille (1) d'un instrument médical générique, une aiguille avec un corps cylindrique ayant un premier diamètre et comportant une partie agrandie (12) ayant un diamètre plus grand que le premier diamètre du corps cylindrique étant montée sur ledit instrument médical générique, ledit dispositif (1) comprend : un récipient (2) comportant des trous traversants concentriques (3, 3') adaptés pour permettre à l'aiguille (A) de coulisser dans le récipient (2), un moyen de protection (4) reçu de manière mobile dans le récipient (2), ledit moyen de protection (4) comportant une base (B) ayant un trou (B') avec un diamètre tel que le corps cylindrique de l'aiguille (A) ayant le premier diamètre peut coulisser à travers le trou (B'), et deux éléments de verrouillage (5, 5') s'étendant l'un à l'opposé de l'autre à partir de la base (B), les éléments de verrouillage (5, 5') étant définis par deux mâchoires flexibles lors d'une compression et comportant chacune un sommet (P, P') et une saillie (11, 11') sous la forme d'une ailette ; les éléments de verrouillage (5, 5') étant adaptés pour recouvrir l'extrémité coupante de l'aiguille (A), **caractérisé par le fait que** ledit dispositif (1) peut être activé, au moyen d'un déplacement axial du moyen de protection (4) par rapport au récipient (2), d'un état non-fonctionnel I, dans lequel l'aiguille (A) est librement mobile dans le récipient (2), à un état fonctionnel II, dans lequel lesdits éléments de verrouillage (5, 5') interagissent avec le récipient (2) de façon à : coincer lesdites saillies (11, 11') dans une cavité du récipient (2) positionnée entre les deux trous traversants (3, 3') pour empêcher le dispositif de repasser de l'état fonctionnel II à l'état non-fonctionnel I et pour recouvrir l'extrémité coupante (A') de l'aiguille ;
- le diamètre du trou (B') de la base (B) est plus petit que le diamètre de la partie agrandie (12), de telle sorte que, lorsque le dispositif de protection (1) est déplacé par rapport à l'aiguille pour recouvrir l'extrémité coupante de l'aiguille (A), la partie de diamètre agrandi (12) peut entrer en contact avec la base (B) pour tirer le moyen de protection (4) axialement par rapport au récipient (2) ;
- chaque ailette est dimensionnée et formée pour se projeter à partir de la mâchoire respective dans une mesure telle que, dans l'état non-fonctionnel I, chaque extrémité libre de l'ailette vient en butée contre une paroi interne du récipient pour courber les mâchoires et déplacer les sommets (P, P') à l'écart l'un de l'autre d'une distance qui permet à l'aiguille A de se déplacer entre eux sans frottement, et chaque mâchoire (15, 15') est configurée pour revenir élastiquement à une position dans laquelle son sommet (P, P') recouvre l'extrémité coupante de l'aiguille (A) lorsque sa saillie est coincée dans la cavité du récipient (2) dans l'état fonctionnel II.

2. Dispositif de protection d'aiguille d'un instrument médical générique selon la revendication 1, **caractérisé par le fait que** le moyen de protection (4) est une plaque métallique.

3. Dispositif de protection d'aiguille (1) d'un instrument médical générique selon l'une des revendications précédentes, **caractérisé par le fait que** les éléments de verrouillage (5, 5') sont deux mâchoires flexibles (15, 15') comportant chacune un sommet incurvé vers l'intérieur (P, P').

4. Dispositif de protection d'aiguille (1) d'un instrument médical générique selon l'une quelconque des revendications précédentes, **caractérisé par le fait que** les éléments de verrouillage (5, 5') ont une longueur différente l'une de l'autre.

5. Dispositif de protection d'aiguille (1) d'un instrument médical générique selon la revendication 1, **caractérisé par le fait que** lesdites saillies (11 ; 11') comprennent deux ailettes espacées et positionnées à des bords transversalement externes desdits éléments de verrouillage (5, 5').

6. Dispositif de protection d'aiguille (1) d'un instrument médical générique selon la revendication 5, **caractérisé par le fait que** lesdites deux ailettes sont positionnées à une distance prédéterminée d'une base du moyen de protection (4) à au moins 40 % de la hauteur desdits éléments de verrouillage (5 ; 5').

7. Dispositif de protection d'aiguille (1) d'un instrument médical générique selon la revendication 5, **caractérisé par le fait que** chaque ailette est reliée de manière rigide à l'extrémité axialement externe d'un élément de verrouillage (5, 5') et se projette sensiblement perpendiculairement à partir de celle-ci.

8. Dispositif de protection d'aiguille (1) d'un instrument médical générique selon l'une quelconque des revendications précédentes, **caractérisé par le fait qu'**il comprend au moins un élément de recouvrement (30), positionné entre une saillie et un sommet incurvé (P ; P') dudit élément de verrouillage (5, 5') pour empêcher l'aiguille de sortir latéralement dans l'état fonctionnel (II).

9. Dispositif de protection d'aiguille (1) d'un instrument médical générique selon la revendication 8, **caractérisé par le fait qu'**il comprend deux éléments de recouvrement espacés (30), chacun étant positionné à l'extrémité transversalement externe d'un élément de verrouillage (5 ; 5') entre une saillie et un sommet incurvé (P, P') dudit élément de verrouillage (5, 5') pour empêcher l'aiguille de sortir latéralement dans l'état fonctionnel (II).

10. Dispositif de protection d'aiguille (1) d'un instrument médical générique selon la revendication 8 ou 9, **caractérisé par le fait que** chaque élément de recouvrement (30) est relié de manière rigide à une ailette.

11. Dispositif de protection d'aiguille (1) d'un instrument médical générique selon l'une quelconque des revendications précédentes, **caractérisé par le fait que** le récipient (2) comprend au moins trois cavités concentriques cylindriques (C1, C2, C3) adjacentes les unes aux autres.

12. Dispositif de protection d'aiguille (1) d'un instrument médical générique selon la revendication 11, **caractérisé par le fait que** deux (C1, C3) des trois cavités (C1, C2, C3) ont un même diamètre et **par le fait que** la cavité (C2) positionnée entre les deux extrémités (C1, C3) a un diamètre plus grand que les diamètres des cavités positionnées aux deux extrémités.

13. Dispositif de protection d'aiguille (1) d'un instrument médical générique selon la revendication 12, **caractérisé par le fait qu'**au moins une cavité a une fente (101) pour recevoir au moins partiellement le moyen de protection (4).

14. Dispositif de protection d'aiguille (1) d'un instrument médical générique selon l'une des revendications précédentes, **caractérisé par le fait que**, dans l'état non-fonctionnel (I), la distance entre les sommets incurvés (P, P') est plus grande que le diamètre de l'aiguille (A).

15. Dispositif de protection d'aiguille (1) d'un instrument médical générique selon l'une des revendications précédentes, dans lequel l'aiguille (A) est montée sur un cathéter veineux.
